# EUROPÄISCHE PATENTSCHRIFT

(11) **EP 0 460 474 B1**
(45) Veröffentlichungstag und Bekanntmachung des Hinweises auf die Patenterteilung: **27.12.2000**
(21) Anmeldenummer: 91108503.3
(22) Anmeldetag: 25.05.1991
(51) Int. Cl.: C07D 207/267

(54) **Verfahren zur Herstellung von N-substituierten Pyrrolidonen**
Process for the preparation of N-substituted pyrrolidones
Procédé pour la préparation de pyrrolidones N-substitués

(30) Priorität: 07.06.1990 DE 4018243
(43) Veröffentlichungstag der Anmeldung: 11.12.1991
(73) Patentinhaber: BASF Aktiengesellschaft, 67063 Ludwigshafen (DE)
(72) Erfinder: Weyer, Hans-Jürgen, Dr., W-6800 Mannheim 1 (DE); Fischer, Rolf, Dr., W-6900 Heidelberg (DE); Harder, Wolgang, Dr., W-6940 Weinheim (DE)

(56) Entgegenhaltungen:
- EP-A- 0 304 696
- US-A- 3 109 005
- US-A- 4 731 454

## Beschreibung

Die vorliegende Erfindung betrifft ein Verfahren zur Herstellung von N-substituierten Pyrrolidonen durch die katalytische Hydrierung von Maleinsäureanhydrid, Maleinsäure und/oder Fumarsäure in Gegenwart eines primären Amins und eines Lösungsmittels, bei erhöhter Temperatur und bei erhöhtem Druck.

Im folgenden Text werden die nachstehenden Abkürzungen benutzt: MSA für Maleinsäureanhydrid, BSA für Bernsteinsäureanhydrid, MS für Maleinsäure, FS für Fumarsäure, BS für Bernsteinsäure und NMP für N-Methylpyrrolidon.

Aus zahlreichen Patentanmeldungen ist bekannt, BSA-Amin-Mischungen, N-substituierte Maleinimide oder Bernsteinsäureimide in Gegenwart bestimmter, unterschiedlicher Katalysatorsysteme zu N-substituierten Pyrrolidonen zu hydrieren. Alle diese Verfahren haben den Nachteil, daß die genannten Einsatzstoffe in einer separaten Umsetzung, beispielsweise aus MSA, erzeugt werden müssen. Infolgedessen sind diese Verfahren unwirtschaftlich und ohne Bedeutung für die industrielle Herstellung von N-substituierten Pyrrolidonen.

Hingegen sind nur wenige Verfahren bekannt, welche die Direktsynthese von N-substituierten Pyrrolidonen aus MSA, MS und/oder FS ermöglichen:

US-A 3 109 005 beschreibt ein Verfahren zur Direktsynthese von N-Methylpyrrolidon (NMP) durch die Hydrierung von MSA-Methylamin-Gemischen an einem Raney-Nickel-Katalysator, bei einer Temperatur von 270°C, einem Druck von 250 bar und in Dioxan als Lösungsmittel. Bei Reaktionszeiten von 10 h werden nach diesem Verfahren NMP-Ausbeuten in Höhe von 70 % erzielt.

DE-A 2 20 600 beschreibt die Hydrierung von MSA-Methylamin-Mischungen zu NMP an Palladium-Trägerkatalysatoren. Nach diesem Verfahren erhält man unter den günstigsten Bedingungen, nämlich einer Temperatur von 275°C, einem Druck von 120 bar und Wasser als Lösungsmittel, eine Ausbeute von 44 %. Anmerkenswert ist der Befund, daß unter fast identischen Reaktionsbedingungen im Reaktionssystem MSA-Ammoniak-Wasser 2-Pyrrolidon-Ausbeuten von bis zu 78 % erzielbar sind. Demnach ist NMP nach dieser Verfahrensweise offensichtlich schwerer und in niedrigeren Ausbeuten zugänglich als 2-Pyrrolidon.

US-A 4 731 454 beschreibt ein Verfahren zur Herstellung von N-substituierten 2-Pyrrolidonen, in dem vorgebildete Imide, Amide oder Ammoniumsalze der Maleinsäure, Fumarsäure oder Bernsteinsäure an einem Cobalt-Katalysator, der zusätzlich mindestens ein Element, ausgewählt aus der Gruppe bestehend aus Molybdän, Wolfram und Rhenium, enthält, hydriert werden.

EP-A 304 696 betrifft ein Verfahren zur Herstellung von 1,4-Butandiol und/oder Tetrahydrofuran durch katalytische Hydrierung von Maleinsäureanhydrid, Bernsteinsäureanhydrid, Maleinsäure, Bernsteinsäure, Fumarsäure oder den Alkylestern dieser Säuren bei Temperaturen von 100 bis 350°C und Drücken von 50 bis 350 bar an einem Katalysator, der als katalytisch aktives Metall Cobalt und mindestens eines der Elemente Kupfer und Phosphor enthält, wobei man die Hydrierung gegebenenfalls in Gegenwart eines aliphatischen Alkohols vornimmt.

Aufgrund dieser mäßigen oder ungenügenden Ausbeuten sind diese Verfahren gegenüber dem herkömmlichen Verfahren zur NMP-Herstellung, das ist die Umsetzung von γ-Butyrolacton mit Methylamin, nicht konkurrenzfähig und haben keine technische Bedeutung erlangt.

N-substituierte Pyrrolidone, insbesondere NMP, werden in großen Mengen als Lösungs- und Extraktionsmittel verwendet und produziert. MSA ist eine in großen Mengen verfügbare, preiswerte Grundchemikalie. Es bestand daher die Aufgabe, ein wirtschaftliches Verfahren zur Direktsynthese von N-substituierten Pyrrolidonen durch die katalytische Hydrierung von MSA(I)-Amin-Mischungen zu entwickeln. Insbesondere sollte ein Katalysator zur Verfügung gestellt werden, der die Herstellung der genannten Pyrrolidone in guten Ausbeuten ermöglicht und sich durch eine hohe Katalysatorstandzeit auszeichnet.

Dementsprechend wurde ein Verfahren zur Herstellung von N-substituierten Pyrrolidonen der Formel II 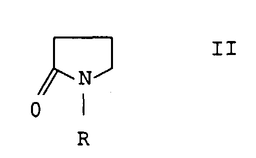 in der R eine aliphatische Gruppe mit 1 bis 10 Kohlenstoffatomen oder eine cycloaliphatische Gruppe mit 5 bis 8 Kohlenstoffatomen ist, durch die katalytische Hydrierung von Maleinsäureanhydrid, Maleinsäure und/oder Fumarsäure mittels eines Cobalt- und Molybdän-haltigen Katalysators in Gegenwart eines primären aliphatischen Amins R-NH₂ mit 1 bis 10 Kohlenstoffatomen oder eines primären cycloaliphatischen Amins mit 5 bis 8 Kohlenstoffatomen und eines Lösungsmittels bei Temperaturen von 100 bis 350°C und bei einem Druck von 50 bis 350 bar, wobei man einen Katalysator verwendet, dessen aktive Masse zu mindestens 40 Gew.-% aus Cobalt (ber. als Co) besteht und als weitere aktive Bestandteile 3 bis 10 Gew.-% Mangan (ber. als Mn), 0,1 bis zu 20 Gew.-% Phosphorsäure, 0,01 bis zu 1 Gew.-% Natrium (ber. als Na), 12 bis 30 Gew.-% Kupfer (ber. als Cu) und 1 bis 5 Gew.-% Molybdän (ber. als Mo) enthält und ein Molverhältnis von Maleinsäureanhydrid, Maleinsäure und/oder Fumarsäure zu Amin von 1:0,5 bis 1:2 wählt.

Die den erfindungsgemäßen Verfahren zugrundeliegende Hydrierung von MSA(I)-Amin-Mischungen zu N-substituierten Pyrrolidonen I) läßt sich formal durch die folgende Reaktionsgleichung beschreiben

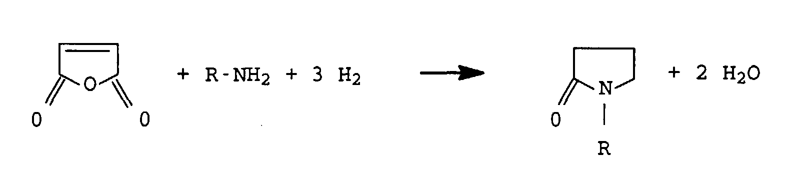

Als primäre Amine R-NH₂ können aliphatische Amine mit 1 bis 10, vorzugsweise 1 bis 4 Kohlenstoffatomen oder primäre cycloaliphatische Amine mit 5 bis 8 Kohlenstoffatomen eingesetzt werden. Beispielhaft seien die folgenden Amine als Reaktionspartner von MSA aufgezählt: Methylamin, Propylamin, Butylamin, Hexylamin, Decylamin, Cyclopentylamin, Cyclohexylamin. Besonders bevorzugt ist die Umsetzung mit Methylamin.

Der Verwendung von MSA als Einsatzstoff im erfindungsgemäßen Verfahren ist die Verwendung von MS und/oder FS äquivalent. Alle diese Einsatzstoffe können in fester, flüssiger oder gasförmiger Form in das erfindungsgemäße Verfahren eingebracht werden. Besonders bevorzugt ist die Verwendung von MSA. Vorteilhafterweise wird gasförmiges MSA, wie es in der Regel im technischen Maßstab bei der katalytischen Oxidation von Butan, Buten oder aromatischen Kohlenwasserstoffen gebildet wird, in einem Lösungsmittel absorbiert und die erhaltene Lösung ohne weitere Aufarbeitung der hydrierenden Umsetzung zugeführt.

Im erfindungsgemäßen Verfahren werden solche Katalysatoren angewandt, deren katalytisch aktive Masse zu mindestens 40 Gew.% aus Cobalt (ber. als Co) besteht und als weitere aktive Bestandteile 3 bis 10 Gew.-%, vorzugsweise 3 bis 7 Gew.-% Mangan (ber. als Nm), 0,1 bis 20 Gew.-%, vorzugsweise 0,1 bis 5 Gew.-% Phosphorsäure, 0,01 bis 1 Gew.-%, vorzugsweise 0,01 bis 0,5 Gew.-% Natrium (ber. als Na), 12 bis 30 Gew.-%, vorzugsweise 12 bis 18 Gew.-% Kupfer (ber. als Cu) und 1 bis 5 Gew.-%, vorzugsweise 1 bis 4 Gew.-% Molybdän (ber. als Mo) enthält.

Die erfindungsgemäß verwendeten Katalysatoren können im erfindungsgemäßen Verfahren sowohl als Trägerkatalysatoren oder vorzugsweise im kompakter Form, d.h. ohne Träger eingesetzt werden. Die Art des Trägermaterials ist in der Regel nicht kritisch, es können übliche Trägermaterialien wie Siliciumdioxid, Aluminiumoxide, Titandioxide, Aktivkohle, Silikate oder Zeolithe verwendet werden. Erforderlichenfalls können zur Herstellung der Katalysatoren Bindemittel oder Formhilfsmittel mitverwendet werden.

Die Katalysatoren werden vorzugsweise vor ihrem Einsatz im erfindungsgemäßen Verfahren mit Wasserstoff aktiviert. Dabei werden die nach der Calcinierung im allgemeinen in Form ihrer Oxide vorliegenden aktiven Katalysatorbestandteile größtenteils reduziert und zwar in der Regel zu den entsprechenden Metallen. Weitere Einzelheiten zu den Katalysatoren und ihrer Herstellung können DE-A 23 21 201 sowie der DE-A-39 04 083 entnommen werden.

Die Katalysatoren können im erfindungsgemäßen Verfahren in suspendierter Form angewandt werden, bevorzugt ist jedoch eine Festbettanordnung des Katalysators, über die die Einsatzstoffe in der Sumpf- oder vorzugsweise der Rieselfahrweise geleitet werden.

Die erfindungsgemäße hydrierende Umsetzung der Einsatzstoffe MSA und primäres Amin wird in Gegenwart eines Lösungsmittels ausgeführt. als Lösungsmittel können praktisch alle Lösungsmittel verwendet werden, welche sich unter den Bedingungen der Hydrierung inert verhalten, beispielsweise Wasser, aliphatische und aromatische Kohlenwasserstoffe oder Ether wie Diethylether, Diisopropylether, Methyl-tert.-Butylether, Dioxan oder Tetrahydrofuran oder Gemische dieser Lösungsmittel. Vorteilhaft ist die Benutzung von N-substituierten Pyrrolidonen als Lösungsmittel, wie sie bei der erfindungsgemäßen Umsetzung entstehen.

Besonders bevorzugt ist das Lösungsmittel Wasser, insbesondere für die Herstellung von NMP.

Die Zugabereihenfolge von MSA, MS oder FS und primärem Amin zur Reaktionsmischung ist im allgemeinen nicht kritisch.

MSA, MS oder FS werden in der Regel bezüglich des Lösungsmittels in einem Molverhältnis von 1 : 1 bis 1 : 100, vorzugsweise in einem Molverhältnis von 1 : 5 bis 1 : 50 eingesetzt.

Bezüglich des primären Amines werden MSA, MS oder FS im erfindungsgemäßen Verfahren in einem Molverhältnis von 1 : 0,5 bis 1 : 2, vorzugsweise von 1 : 0,8 bis 1 : 1,5 und besonders bevorzugt in einem Molverhältnis von 1 : 1 bis 1 : 1,3 angewandt.

Der Wasserstoff kann der Umsetzung in stöchiometrischen, vorzugsweise in überschüssigen Mengen zugeführt werden. Die Menge des zugeführten überschüssigen Wasserstoffs ist an sich nicht kritisch, da der überschüssige, unverbrauchte Wasserstoff in die Umsetzung zurückgeführt oder gewünschtenfalls auch abgefackelt werden kann.

Da die hydrierende Umsetzung von MSA, MS oder FS mit primären Aminen und Wasserstoff zu N-substituierten Pyrrolidonen über eine Vielzahl von Intermediaten, wie z.B. Maleinamide, Maleinimide, BSA, BS, Bernsteinsäureimide oder Bernsteinsäureamide, verläuft, liegt es auf der Hand, daß auch diese Intermediate als Einsatzstoffe in die erfindungsgemäße, hydrierende Umsetzung eingebracht werden können. Es ist selbstverständlich, daß ein solches Vorgehen dem erfindungsgemäßen Verfahren äquivalent ist.

Die Umsetzung wird bei Temperaturen von 100 bis 350°C, vorzugsweise von 150 bis 300°C und insbesondere von 180 bis 280°C vorgenommen. Dabei wird bei Drucken von 50 bis 350 bar und bevorzugt, bei 100 bis 300 bar gearbeitet.

Die Umsetzung kann diskontinuierlich, beispielsweise in Rührautoklaven, erfolgen, vorzugsweise wird aber die kontinuierliche Fahrweise, z.B. in Rohrreaktoren oder Rohrbündelreaktoren, gewählt, wobei die Hydrierwärme durch Außen- oder Innenkühlung abgeführt werden kann. Eine weiter Möglichkeit zu Regulierung der Reaktionstemperatur besteht in der Rückführung eines Teils des Hydrieraustrages sowie des überschüssigen Wasserstoffs nach vorhergegangener Abkühlung, beispielsweise in einem Wärmetauscher.

Die hydrierende Umsetzung kann so gesteuert werden, daß während der gesamten Umsetzungsdauer ein bestimmter Druck- und Temperaturbereich eingehalten wird. Es kann sich aber, insbesondere beim Drucksatz größerer Mengen, positiv für die Selektivität und die Standzeit des Katalysators auswirken, wenn man die Umsetzung auf verschiedenen Druck- und Temperaturniveaus durchführt, beispielsweise indem man zunächst in einem ersten Reaktor bei Temperaturen von 100 bis 220°C und Drucken von 50 bis 200 bar teilhydriert und anschließend den Hydrieraustrag ohne Aufarbeitung in den nächsten Reaktor leitet, um dort die hydrierende Umsetzung z.B. bei Temperaturen von 220 bis 300°C und Drucken von 200 bis 350 bar zu Ende zu führen.

Die so erhaltenen Hydrierausträge können unter Umständen neben den erwünschten N-substituierten Pyrrolidonen noch geringe Mengen an Nebenprodukten wie N-substituierten Bernsteinsäureimiden, Bernsteinsäurediamiden, Bernsteinsäuremonoamiden oder N-substituierten Pyrrolidinen enthalten. Die Aufarbeitung dieser Hydrierausträge kann durch Extraktion oder vorteilhaft destillativ erfolgen. Die so abgetrennten, teilhydrierten Nebenprodukte können, da sie zu N-substituierten Pyrrolidonen hydrierbar sind, zur Erzielung eines vollständigen Umsatzes in die Hydrierzone zurückgeführt werden.

Das erfindungsgemäße Verfahren ermöglicht die wirtschaftliche, industrielle Direktsynthese von N-substituierten Pyrrolidonen aus MSA, MS und/oder FS und primären Aminen. Dabei werden Ausbeuten von mehr als 90 % erhalten.

### Beispiele

Die %-Angaben in den folgenden Beispielen sind Gew.-%.

### Beispiel 1

Die hydrierende Umsetzung wurde in einem Rohrreaktor Länge: 200 mm, Durchmesser: 16 mm), in dem sich 38 g Katalysator in einem Festbett befanden, durchgeführt. Der Reaktor wurde über einen außenliegenden Heizmantel mit Öl auf die Reaktionstemperatur aufgeheizt. Die gasförmigen und flüssigen Einsatzstoffe durchströmten den Reaktor von oben nach unten (Rieselfahrweise). Der Hydrieraustrag wurde auf Raumtemperatur abgekühlt, entspannt und in einem Gas-Flüssigkeits-Abscheider in seine gasförmigen und flüssigen Bestandteile zerlegt.

Es wurde ein Katalysator der folgenden Zusammensetzung verwendet:
63,4 Gew.-% Kobalt, ber. als CoO
18,1 Gew.-% Kupfer, ber. als CuO
6,8 Gew.-% Mangan, ber. als Nm₃Oₙ
3,1 Gew.-% Molybdän, ber. als MoO₃
0,15 Gew.-% Natrium, ber. als Na₂O
3,3 Gew.-% Phosphorsäure (H₃PO₄)

Der Katalysator wurde in Form von 2,5 bis 4 mm großem Splitt eingesetzt und vor Beginn der Hydrierung mit Wasserstoff aktiviert.

Bei einem Gesamtdruck von 200 bar und einer Temperatur von 250°C wurden dem Reaktor 0,15 kg MSA/kg Katalysator und Stunde, 0,07 kg Methylamin/kg Katalysator und Stunde und 0,55 Wasser/kg Katalysator und Stunde, sowie 2500 NL Wasserstoff/kg Katalysator und Stunde zugeführt.

Die Untersuchung des flüssigen Reaktionsaustrages ergab während einer Laufzeit von 9 h eine durchschnittliche NMP-Ausbeute von 91 %, bezogen auf eingesetztes MSA. Weiterhin wurden ca. 2 % Bernsteinsäure-N-Methylamid und Ca. 1 % Bernsteinsäure/n-Methylimid gefunden.

### Beispiel 2

Bei einem Gesamtdruck von 20 bar und einer Temperatur von 210°C wurden dem Reaktor von Beispiel 1 0,1 kg MSA/kg und Stunde, 0,15 kg Cyclohexylamin/kg Katalysator und Stunde, 0,29 kg Dioxan/kg Katalysator und Stunde und 0,29 kg Wasser/kg Katalysator und Stunde sowie 2000 NL Wasserstoff/kg Katalysator und Stunde zugeführt. N-Cyclohexylpyrrolidon wurde in einer gaschromatographisch ermittelten Ausbeute von 51 %, bezogen auf eingesetztes MSA, erhalten.

## Patentansprüche

1. Verfahren zur Herstellung von N-substituierten Pyrrolidonen der Formel II 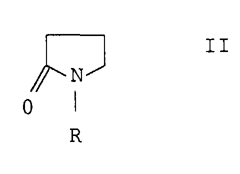 in der R eine aliphatische Gruppe mit 1 bis 10 Kohlenstoffatomen oder eine cycloaliphatische Gruppe mit 5 bis 8 Kohlenstoffatomen ist, durch die katalytische Hydrierung von Maleinsäureanhydrid, Maleinsäure und/oder Fumarsäure mittels eines Cobalt- und Molybdän-haltigen Katalysators in Gegenwart eines primären aliphatischen Amins R-NH₂ mit 1 bis 10 Kohlenstoffatomen oder eines primären cycloaliphatischen Amins mit 5 bis 8 Kohlenstoffatomen und eines Lösungsmittels bei Temperaturen von 100 bis 350°C und bei einem Druck von 50 bis 350 bar, dadurch gekennzeichnet, daß man einen Katalysator verwendet, dessen aktive Masse zu mindestens 40 Gew.-% aus Cobalt (ber. als Co) besteht und als weitere aktive Bestandteile 3 bis 10 Gew.-% Mangan (ber. als Mn), 0,1 bis zu 20 Gew.-% Phosphorsäure, 0,01 bis zu 1 Gew.-% Natrium (ber. als Na), 12 bis 30 Gew.-% Kupfer (ber. als Cu) und 1 bis 5 Gew.-% Molybdän (ber. als Mo) enthält und ein Molverhältnis von Maleinsäureanhydrid, Maleinsäure und/oder Fumarsäure zu Amin von 1:0,5 bis 1:2 wählt.

2. Verfahren nach Anspruch 1, dadurch gekennzeichnet, daß man ein Molverhältnis von Maleinsäureanhydrid zu Lösungsmittel von 1 : 1 bis 1 : 100 einstellt.

3. Verfahren nach Anspruch 1, dadurch gekennzeichnet, daß man als Lösungsmittel Wasser verwendet.

4. Verfahren nach Anspruch 1, dadurch gekennzeichnet, daß man die Hydrierung auf zwei verschiedenen Temperatur- und Druckniveaus durchführt, und dabei zunächst bei Temperaturen von 100 bis 200°C und bei einem Druck von 50 bis 200 bar arbeitet und anschließend die Hydrierung bei Temperaturen von 220 bis 350°C und einem Druck von 200 bis 350 bar zu Ende führt.

5. Verfahren nach Anspruch 1, dadurch gekennzeichnet, daß man als Amin Methylamin einsetzt.

6. Verfahren nach Anspruch 1, dadurch gekennzeichnet, daß man die bei der hydrierenden Umsetzung von Maleinsäureanhydrid, Maleinsäure oder Fumarsäure mit primären Aminen und Wasserstoff entstehenden Intermediate als Einsatzstoffe in die Umsetzung einbringt.

## Claims

1. A process for preparing N-substituted pyrrolidones of the formula II 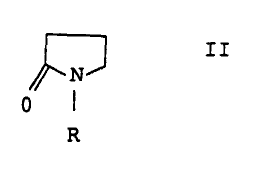 where R is an aliphatic group having from 1 to 10 carbon atoms or a cycloaliphatic group having from 5 to 8 carbon atoms, by catalytic hydrogenation of maleic anhydride, maleic acid and/or fumaric acid, using a cobalt- and molybdenum-containing catalyst in the presence of a primary aliphatic amine R-NH₂ having from 1 to 10 carbon atoms or of a primary cycloaliphatic amine having from 5 to 8 carbon atoms and of a solvent at from 100 to 350°C and at a pressure of from 50 to 350 bar, which comprises using a catalyst whose active composition is composed of at least 40% by weight of cobalt (calc. as Co) and comprises, as another active constituents, from 3 to 10% by weight of manganese (calc. as Mn), from 0.1 to 20% by weight of phosphoric acid, from 0.01 to 1% by weight of sodium (calc. as Na), from 12 to 30% by weight of copper (calc. as Cu) and from 1 to 5% by weight of molybdenum (calc. as Mo), and selecting a value of from 1:0.5 to 1:2 for the molar ratio of maleic anhydride, maleic acid and/or fumaric acid to amine.

2. A process as claimed in claim 1, wherein a value of from 1:1 to 1:100 is set for the molar ratio of maleic anhydride to solvent.

3. A process as claimed in claim 1, wherein the solvent used comprises water.

4. A process as claimed in claim 1, wherein the hydrogenation is carried out at two different levels of temperature and pressure, first operating at from 100 to 200°C and at a pressure of from 50 to 200 bar, then completing the hydrogenation at from 220 to 350°C and at a pressure of from 200 to 350 bar.

5. A process as claimed in claim 1, wherein the amine used comprises methylamine.

6. A process as claimed in claim 1, wherein the intermediates arising during the hydrogenation reaction of maleic anhydride, maleic acid or fumaric acid with primary amines and hydrogen are introduced into the reaction as starting materials.

## Revendications

1. Procédé de préparation de pyrrolidones N-substituées de formule II 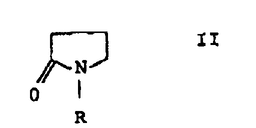 dans laquelle R est un groupe aliphatique ayant 1 à 10 atomes de carbone ou un groupe cycloaliphatique ayant 5 à 8 atomes de carbone, par l'hydrogénation catalytique de l'anhydride maléique, l'acide maléique et/ou l'acide fumarique au moyen d'un catalyseur contenant du cobalt et du molybdène en présence d'une amine primaire aliphatique R-NH₂ ayant 1 à 10 atomes de carbone ou d'une amine primaire cyclo-aliphatique ayant 5 à 8 atomes de carbone et d'un solvant à des températures de 100°C à 350°C et sous une pression de 50 à 350 bar, caractérisé en ce que l'on utilise un catalyseur, dont la masse active est constituée d'au moins 40% en poids de cobalt (calculé en Co) et contient comme autres composants actifs, de 3 à 10% en poids de manganèse (calculé en Mn), de 0,1 jusqu'à 20% d'acide phosphorique, de 0,01 jusqu'à 1% en poids de sodium (calculé en Na), de 12 à 30% en poids de cuivre (calculé en Cu) et de 1 à 5% en poids de molybdène (calculé en Mo) et l'on choisit un rapport molaire de l'anhydride maléique, l'acide maléique et/ou l'acide fumarique à l'amine de 1:0,5 à 1:2.

2. Procédé selon la revendication 1, caractérisé en ce que l'on ajuste un rapport molaire de l'anhydride maléique au solvant de 1:1 à 1:100.

3. Procédé selon la revendication 1, caractérisé en ce que l'on utilise l'eau comme solvant.

4. Procédé selon la revendication 1, caractérisé en ce que l'on réalise l'hydrogénation à deux niveaux de température et de pression différents, et on travaille d'abord à des températures de 100°C à 200°C et sous une pression de 50 à 200 bar puis on poursuit l'hydrogénation jusqu'à achèvement à des températures de 220°C à 350°C et sous une pression de 200 à 350 bar.

5. Procédé selon la revendication 1, caractérisé en ce que l'on met en oeuvre comme amine, la méthylamine.

6. Procédé selon la revendication 1, caractérisé en ce que l'on introduit les produits intermédiaires formés pendant la réaction d'hydrogénation de l'anhydride maléique, l'acide maléique ou l'acide fumarique, dans la réaction en tant que matières de départ.
